# EUROPEAN PATENT SPECIFICATION

(11) **EP 3 453 346 B1**
(45) Date of publication and mention of the grant of the patent: **06.04.2022**
(21) Application number: 18190574.6
(22) Date of filing: 23.08.2018
(51) Int. Cl.: A61B 17/34

(54) **SURGICAL CANNULA ASSEMBLY**
CHIRURGISCHE KANÜLENANORDNUNG
ENSEMBLE DE CANULE CHIRURGICALE

(30) Priority: 24.08.2017 US 201762549590 P; 24.07.2018 US 201816043279
(43) Date of publication of application: 13.03.2019
(73) Proprietor: Covidien LP, Mansfield, MA 02048 (US)
(72) Inventor: BUYDA, Oksana, East Haven, CT 06513 (US); TOKARZ, Christopher, Torrington, CT 06790 (US); ADINOLFI, Amanda M., Wallingford, CT 06492 (US)
(74) Representative: Maschio, Antonio

(56) References cited:
- EP-A1- 2 196 161
- EP-A2- 2 168 626
- US-A1- 2009 221 960
- US-A1- 2017 135 721

## Description

### CROSS-REFERENCE TO RELATED APPLICATIONS

### BACKGROUND

### 1. Field of the Disclosure

The present disclosure relates generally to surgical access devices and more particularly to a surgical cannula assembly for use in a minimally invasive surgical procedure. Such a surgical cannula is known from US2017/135721A1 and US2009/221960A1.

### 2. Description of the Related Art

Minimally invasive surgical procedures including both endoscopic and laparoscopic procedures permit surgery to be performed on organs, tissues, and vessels far removed from an opening within the tissue. In laparoscopic procedures, the abdominal cavity is insufflated with an insufflation gas, e.g., CO₂, to create a pneumoperitoneum thereby providing access to the underlying organs. A laparoscopic instrument is introduced through a cannula accessing the abdominal cavity to perform one or more surgical tasks. An interior of the cannula may include a seal to establish a substantially fluid-tight seal about the instrument to preserve the integrity of the pneumoperitoneum.

While minimally invasive surgical procedures have proven to be quite effective in surgery, several limitations remain. For example, the cannula which is subjected to the pressurized environment, i.e., the pneumoperitoneum, may exhibit a tendency to back out of the incision in the abdominal wall particularly during manipulation of the instrument within the cannula. Conventional cannulas may incorporate an inflatable balloon at the end of the cannula in an effort to resist withdrawal of the cannula from the tissue site.

### SUMMARY

Accordingly, the present disclosure is directed to a surgical cannula as claimed in claim 1. Preferred embodiments of this cannula are claimed in claims 2-13.

In accordance with one embodiment, the surgical cannula assembly includes an elongated cannula member, first and second expandable members mounted to a proximal portion of the cannula member, and a fluid-transfer assembly. The cannula member defines a longitudinal passageway, and the first and second expandable members are in fluid communication with one another. The fluid-transfer assembly includes a proximal flange coupled to the proximal portion of the cannula member, and a distal flange coupled to the proximal portion of the cannula member. The first expandable member is disposed between the proximal and distal flanges such that movement of at least one of the proximal or distal flanges along the cannula member adjusts a pressure applied by the proximal and distal flanges on the first expandable member to transfer an inflation medium between the first and second expandable members.

In embodiments, the proximal flange may be longitudinally movable relative to the cannula member, and the distal flange may be longitudinally fixed relative to the cannula member.

In embodiments, the proximal flange may be attached to a proximally-oriented surface of the first expandable member, and the distal flange may be attached to a distally-oriented surface of the first expandable member.

In embodiments, the proximal and distal flanges may be longitudinally spaced from one another along a longitudinal axis defined by the cannula member.

In embodiments, approximation of the proximal and distal flanges may compress the first expandable member therebetween to transfer a portion of the inflation medium from the first expandable member toward the second expandable member. Expansion of the proximal and distal flanges may transfer the portion of the inflation medium from the second expandable member toward the first expandable member.

In embodiments, the proximal or distal flange may include a locking feature configured to releasably attach to the other of the proximal or distal flange when the proximal and distal flanges are in an approximated position relative to one another. The locking feature may include a first resilient tab extending from a first side of one of the proximal or distal flanges, and a second resilient tab extending from a second side of one of the proximal or distal flanges. The first and second resilient tabs may be configured to releasably engage one of the proximal or distal flanges when the proximal and distal flanges are in the approximated position.

In embodiments, the surgical cannula assembly includes an elongated sleeve interconnecting the first and second expandable members. The sleeve is disposed about the cannula member. Each of the first and second expandable members defines a cavity therein. The sleeve defines a longitudinal channel in fluid communication with the cavity of each of the first and second expandable members.

In embodiments, the first and second expandable members are monolithically formed with respective proximal and distal portions of the sleeve.

In embodiments, the cannula member may include a plurality of longitudinal ribs extending along a longitudinal axis defined by the cannula member. Adjacent longitudinal ribs may define a longitudinal channel therebetween. One or more of the longitudinal channels may fluidly interconnect the first and second expandable members.

In embodiments, the surgical cannula assembly may further include one or more conduits positioned within a respective longitudinal channel of the cannula member. The first expandable member may be in fluid communication with a proximal opening in the conduit, and the second expandable member may be in fluid communication with a distal opening in the conduit.

In embodiments, the surgical cannula assembly may further include a collar and an elongated sleeve. The collar may be mounted adjacent the proximal portion of the cannula member and may define a fluid port configured for coupling to a source of inflation fluids. The sleeve may extend proximally from the second expandable member and be disposed about the cannula member. The sleeve may terminate proximally within the collar.

In embodiments, the cannula member may include a plurality of protuberances disposed both circumferentially about the cannula member and along a longitudinal axis defined by the cannula member. The protuberances may be configured to maintain the sleeve spaced from an outer surface of the cannula member to define a plurality of fluid pathways that fluidly interconnect the fluid port and the second expandable member.

In embodiments, the surgical cannula assembly may further include an annular insert extending along a longitudinal axis defined by the cannula member and received within an annular slot defined between an outer surface of the cannula member and an inner surface of the collar. The annular insert may define a fluid pathway that fluidly interconnects the fluid port and the second expandable member.

In accordance with another embodiment of the present disclosure, a surgical cannula assembly includes an elongated cannula member, an expandable member mounted to a distal portion of the cannula member, and a fluid-transfer assembly. The cannula member defines a longitudinal passageway, and the fluid-transfer assembly includes a cylindrical member and a piston. The cylindrical member is disposed about the proximal portion of the cannula member and defines a cavity. The piston is disposed about the proximal portion of the cannula and within the cavity of the cylindrical member. The cavity of the cylindrical member and an inner chamber of the expandable member are fluidly coupled. Movement of the piston relative to the cylindrical member along the cannula member transfers an inflation medium between the cavity of the cylindrical member and the inner chamber of the expandable member.

In embodiments, the piston may be coupled to the cannula member such that rotation of the piston moves the piston axially relative to the cylindrical member.

In embodiments, the cylindrical member may define an annular recess in an inner surface thereof configured to releasably receive the piston.

In embodiments, the surgical cannula assembly may include a conduit having a proximal portion disposed within the cavity of the cylindrical member, and a distal portion disposed within the inner chamber of the expandable member, such that a portion of the inflation medium is transferred between the cavity of the cylindrical member and the inner chamber of the expandable member via the conduit.

Other features of the present disclosure will be appreciated from the following description.

### BRIEF DESCRIPTION OF THE DRAWINGS

Various aspects and features of the present disclosure are described hereinbelow with references to the drawings, wherein:
FIG. 1 is a perspective view of a surgical cannula assembly of the present disclosure illustrating an elongated cannula member, an outer sleeve and associated proximal and distal expandable members coaxially positioned about the cannula member, and a fluid-transfer assembly coupled to the proximal expandable member;
FIG. 2 is a cutaway view of the surgical cannula assembly of FIG. 1, illustrating an inner chamber of the proximal expandable member of the sleeve and a distal flange of the fluid-transfer assembly;
FIG. 3 is a perspective view of a cross-section, taken along line 3-3 of FIG. 2, of the surgical cannula assembly, illustrating a plurality of longitudinal channels defined between the sleeve and the cannula member;
FIG. 4 is a cross-section, taken along line 4-4 of FIG. 1, of the surgical cannula assembly;
FIG. 5 is a cross-section, taken along line 5-5 of FIG. 1, of the surgical cannula assembly;
FIG. 6 is a longitudinal cross-section of the surgical cannula assembly of FIG. 1 and an obturator extending therethrough, illustrating the surgical cannula assembly and the obturator positioned within an abdominal cavity;
FIG. 7 is a perspective view of another embodiment of a surgical cannula assembly of the present disclosure, illustrating an elongated cannula member, an outer sleeve and associated distal expandable member coaxially positioned about the cannula member, and a fluid-transfer assembly coupled to the cannula member;
FIG. 8 is a cross-section, taken along line 8-8 of FIG. 7, of the fluid-transfer assembly;
FIG. 9 is a cross-sectional view of another embodiment of a fluid-transfer assembly for use with any of the disclosed surgical cannula assemblies;
FIG. 10 is a plan view of yet another embodiment of a surgical cannula assembly of the present disclosure, illustrating an elongated cannula member, an outer sleeve and associated distal expandable member coaxially positioned about the cannula member, and a collar with a fluid port coupled to the cannula member;
FIG. 11 is a plan view of the surgical cannula assembly of FIG. 10, illustrated with the outer sleeve removed;
FIG. 12 is a cross-section, taken along line 12-12 of FIG. 10, of the surgical cannula assembly;
FIG. 13 a cross-section, taken along line 13-13 of FIG. 12, of the surgical cannula assembly;
FIG. 14 is an enlarged view of the area of detail 14 in FIG. 12;
FIG. 15 is a perspective view of another embodiment of a surgical cannula assembly of the present disclosure, illustrating an elongated cannula member, an outer sleeve and associated distal expandable member coaxially positioned about the cannula member, a collar with a fluid port coupled to the cannula member, and a pair of inserts;
FIG. 16 is a cross-section, taken along line 16-16 in FIG. 15, of the surgical cannula assembly;
FIG. 17 is a perspective view of a cross-section, taken along line 17-17 in FIG. 15, of the surgical cannula assembly;
FIG. 18 is a cross-section, taken along line 18-18 in FIG. 15, of the surgical cannula assembly; and
FIG. 19 is a transverse cross-sectional view of another embodiment of an insert for use with any of the disclosed surgical cannula assemblies.

### DETAILED DESCRIPTION

Particular embodiments of the present disclosure are described hereinbelow with reference to the accompanying drawings. However, it is to be understood that the disclosed embodiments are merely examples of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to employ the present disclosure in virtually any appropriately detailed structure.

In general, the present disclosure provides a surgical cannula assembly including a cannula member and a fluid-transfer assembly that facilitates the transfer of inflation media between an expandable member disposed at a proximal portion of the cannula member and another expandable member disposed at a distal portion of the cannula member. The fluid-transfer assembly may include a pair of flanges or plates that sandwich the proximal expandable member therebetween. Due to the fluid-transfer assembly being a closed fluid system, an approximation of the flanges compresses the proximal expandable member, thereby transferring the inflation medium from the proximal expandable member toward the distal expandable member. To decrease the diameter of the distal expandable member, the flanges are expanded to decompress the proximal expandable member, thereby drawing the inflation medium away from the distal expandable member and back toward the proximal expandable member. Alternate designs of the fluid-transfer assembly are disclosed herein. In other embodiments, the surgical cannula assembly may further include associated structure that enhances the structural stability of the cannula member while defining channels through which inflation media may flow to the internal volume of the distal expandable member.

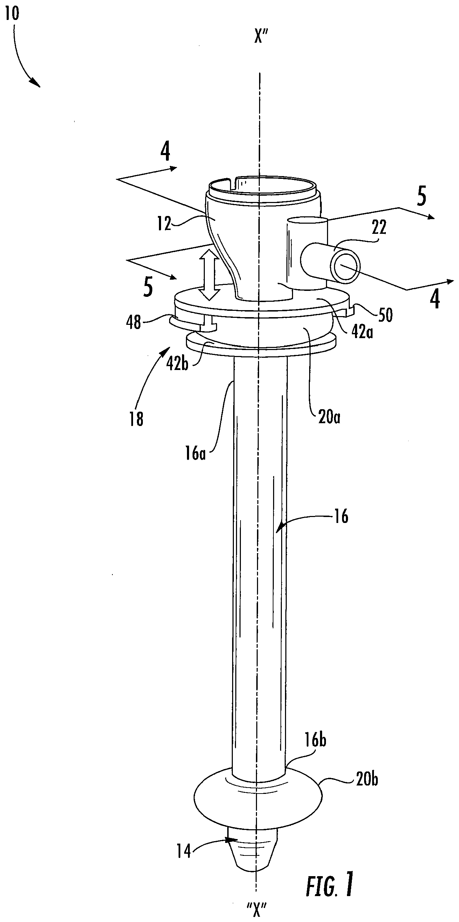

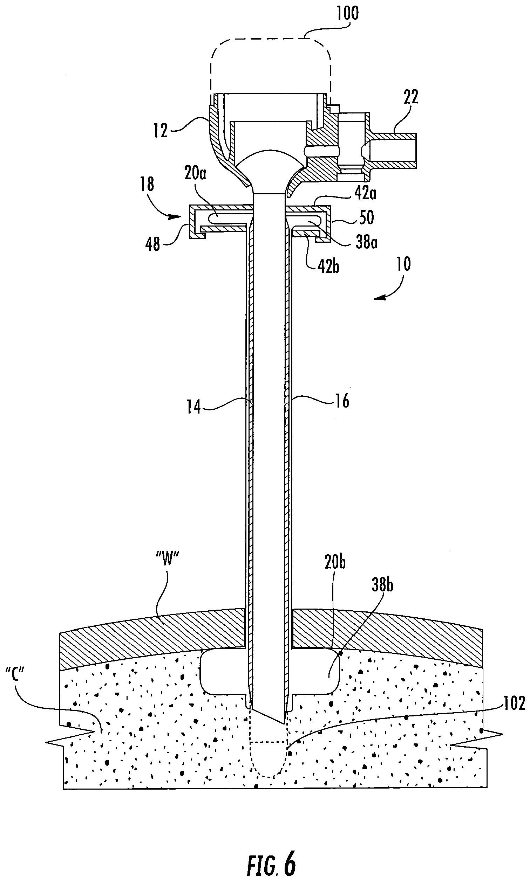

Referring initially to FIG. 1, a surgical cannula assembly of the present disclosure is illustrated. The surgical cannula assembly 10 is intended to permit access to an insufflated abdominal cavity during a laparoscopic procedure to permit the introduction of a surgical object for performing various surgical tasks on internal organs within the cavity. The surgical object may be a surgical instrument such as laparoscopic or endoscopic clip appliers, graspers, dissectors, retractors, staplers, laser probes, photographic devices, tubes, endoscopes and laparoscopes, electro-surgical devices and the like. An obturator 100 (FIG. 6) may be positioned in the surgical cannula assembly 10 to facilitate access to the abdominal cavity. The obturator 100 may be any conventional obturator 100 having a penetrating tip 102 configured to penetrate tissue.

With continued reference to FIG. 1, the surgical cannula assembly 10 includes a cannula housing 12, an elongated cannula member 14 extending distally from the cannula housing 12, an outer sleeve 16 coaxially mounted over the cannula member 14, and a fluid-transfer assembly 18 coupled to the outer sleeve 16 for transferring inflation media between proximal and distal expandable members or balloons 20a, 20b formed with the sleeve 16. The cannula housing 12 is dimensioned for engagement by the clinician and may include one or more internal seals (not shown) adapted to establish a seal about a surgical object introduced therethrough. The cannula housing 12 also may include an insufflation connector 22 (e.g., a luer connector) for connecting to a source of insufflation fluids (not shown) for delivery within, e.g., the abdominal cavity.

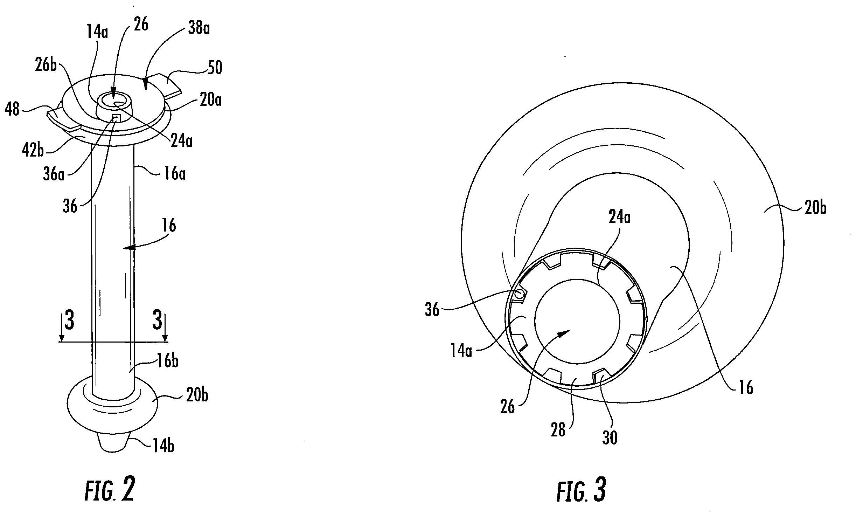

Referring now to FIGS. 1-5, the cannula member 14 has proximal and distal portions 14a, 14b, and defines a longitudinal axis "X" along which the cannula member 14 extends. The cannula member 14 has an inner surface 24a defining a longitudinal passageway 26 to permit passage of the surgical object, and an outer surface 24b. The longitudinal passageway 26 is also in fluid communication with the insufflation connector 18 to convey insufflation fluids into the abdominal cavity to establish and/or maintain the pneumoperitoneum.

The cannula member 14 includes a plurality of longitudinal ribs 28 extending along a majority of the length of its outer surface 24b. In embodiments, the longitudinal ribs 28 are in parallel relation to the longitudinal axis "X." The longitudinal ribs 28 may be configured to enhance the structural stability of the cannula member 14. Circumferentially adjacent longitudinal ribs 28 define longitudinal channels 30 therebetween which also extend along a majority of the length of the cannula member 14 and in parallel relation to the longitudinal axis "X." In the alternative, the longitudinal ribs 28 and the longitudinal channels 30 may be obliquely arranged relative to the longitudinal axis "X" or may have one or more curvatures. The longitudinal channels 30 may be arranged about the longitudinal axis "X" in equidistant circumferentially spaced relation.

The cannula member 14 includes a conduit 36 positioned within at least one longitudinal channel 30 of the cannula member 14. The conduit 36 may be a tube-like structure defining a passage for conveying inflation media. In embodiments, a conduit 36 is disposed in more than one longitudinal channel 30, and, in some embodiments, a conduit 36 is positioned within each longitudinal channel 30. Each conduit 36 defines a proximal opening 36a and a distal opening 36b at respective proximal and distal ends thereof. The proximal opening 36a of the conduit 36 is received within the proximal expandable member 20a, and the distal opening 36b of the conduit 36 is received within the distal expandable member 20b to fluidly interconnect the proximal and distal expandable members 20a, 20b.

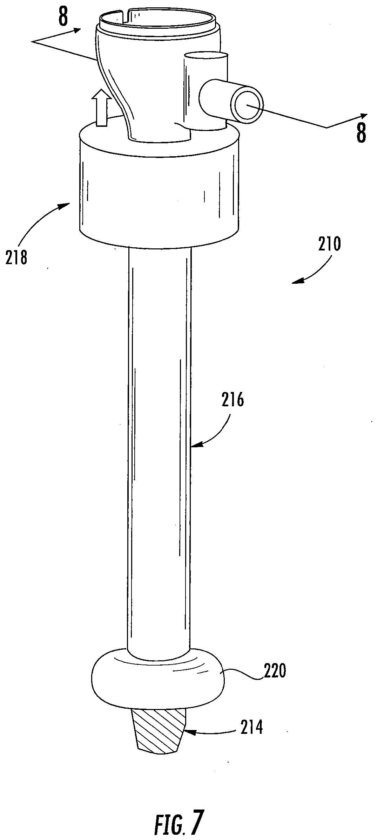

The outer sleeve 16 is coaxially mounted about the cannula member 14 and extends from a position adjacent the cannula housing 12 to a position adjacent the distal portion 24 (FIG. 7) of the cannula member 14. The outer sleeve 16 encloses the conduits 36 within the longitudinal channels 30. The outer sleeve 16 may be secured to the cannula member 14 through a friction or interference fit or with the use of adhesives, cements or the like. In one embodiment, the outer sleeve 16 is fabricated from an elastomeric material such as silicone rubber, polyurethane, polyester or the like. In this embodiment, the outer sleeve 16 may define a diameter approximating the diameter of the cannula member 14 to enclose the longitudinal channels 30 and the conduits 36 in frictional sealing relation therewith. Alternatively, the outer sleeve 16 may have a diameter less than the diameter of the cannula member 14, and is stretched to be positioned about the cannula member 14. Once positioned on the cannula member 14, the outer sleeve 16 is fixed from longitudinal movement, and serves as, e.g., an enclosure enclosing, and optionally sealing, the cannula member 14, the longitudinal ribs 28 and the longitudinal channels 30.

The proximal and distal expandable members 20a, 20b are coupled to respective proximal and distal portions 16a, 16b of the outer sleeve 16 and are coaxially mounted about proximal and distal portions 14a, 14b of the cannula member 14, respectively. The expandable members 20a, 20b may be secured to the respective proximal and distal portions 16a, 16b of the sleeve 16 through any of the aforementioned methodologies discussed hereinabove in connection with the outer sleeve 16. In an embodiment, the expandable members 20a, 20b are secured relative to the sleeve 16 through an interference or friction fit. The expandable members 20a, 20b may be separate components from the outer sleeve 16 coupled thereto by conventional methodologies. In one embodiment, the expandable members 20b, 20b are monolithically formed with the outer sleeve 16 from, e.g., a suitable elastomeric material. For example, the expandable members 20a, 20b may be segments of the outer sleeve 16, which are subjected to a molding or thermoforming process to be capable of transitioning between unexpanded and expanded conditions.

Each of the proximal and distal expandable members 20a, 20b defines a cavity or inner chamber 38a, 38b therein, and the sleeve 16 defines a longitudinal channel 40 in communication with the cavity 38a, 38b of each of the proximal and distal expandable members 20a, 20b. The sleeve 16, together with the expandable members 20a, 20b, defines a closed internal volume that contains a fixed amount of inflation media therein. As such, the proximal and distal expandable members 20a, 20b one of expand radially outwardly or retract radially inward upon passage of inflation media (e.g., air, water, gas, etc.) therebetween via the conduits 36. In one embodiment, the surgical cannula assembly 10 may be devoid of the sleeve 16, and instead a seal is formed at the proximal and distal ends of the conduits 36 to fluidly interconnect the expandable members 20a, 20b.

In one embodiment, the distal expandable member 20b may have perforations (not shown) that are small enough to prevent the leakage of medicament, medical glue, etc. stored within the distal expandable member 20b when the distal expandable member 20b is in the deflated and semi-inflated states. Upon fully inflating the distal expandable member 20b, the perforations will expand to release the medicament or glue from the distal expandable member 20b.

With reference to FIGS. 1, 4, and 5, the fluid-transfer assembly 18 of the surgical cannula assembly 10 will now be described. The fluid-transfer assembly 18 is disposed about the proximal portion 16a of the sleeve 16 and includes proximal and distal flanges 42a, 42b. The proximal and distal flanges 42a, 42b are disc-like members defining central openings through which the cannula member 14 extends. In embodiments, the flanges 42a, 42b may assume any suitable shape, such as, for example, squared, triangular, scalloped, undulating, trapezoidal, or the like. The proximal flange 42a may be attached to a proximally-oriented upper surface 44 of the proximal expandable member 20a, and the distal flange 42b may be attached to a distally-oriented bottom surface 46 of the proximal expandable member 20a, such that the proximal expandable member 20a is captured between the proximal and distal flanges 42a, 42b of the fluid-transfer assembly 18.

The proximal flange 42a is longitudinally movable relative to the proximal portion 14a of the cannula member 14, whereas the distal flange 42b is longitudinally fixed relative to the proximal portion 14a of the cannula member 14. Alternatively, both of or either one of the proximal and distal flanges 42a, 42b may be movable relative to the cannula member 14. The proximal and distal flanges 42a, 42b are longitudinally spaced from one another along the longitudinal axis "X" of the cannula member 14 by a distance equal to a thickness of the proximal expandable member 20a in any of its inflation states. Upon the proximal and distal flanges 42a, 42b being spaced a maximum longitudinal distance from one another (FIG. 1), the proximal expandable member 20a is able to be expanded to its fully inflated state, and upon the proximal and distal flanges 42a, 42b being moved to an approximated position (FIG. 6), the proximal expandable member 20a is able to be deflated to its fully deflated state, as will be described in greater detail below.

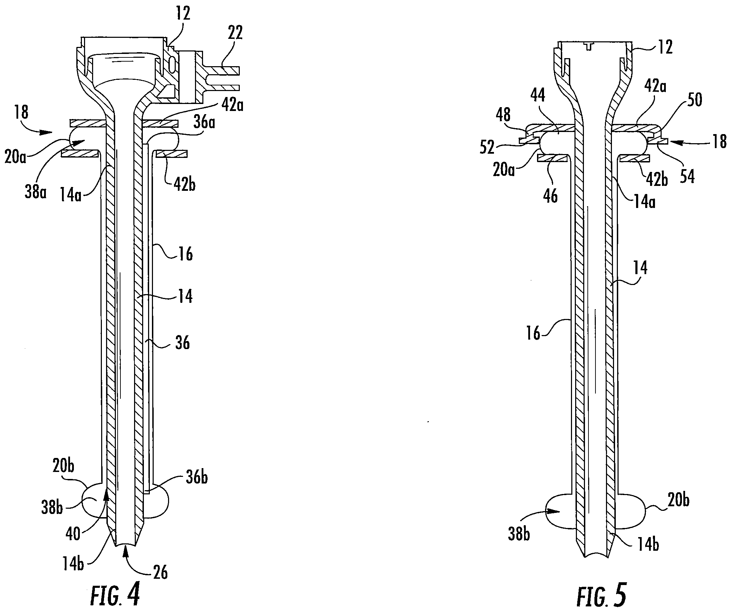

With continued reference to FIG. 5, the proximal flange 42a of the fluid-transfer assembly 18 includes a locking feature configured to releasably attach the proximal flange 42a to the distal flange 42b upon the fluid-transfer assembly 18 moving to the approximated position. The locking feature includes a pair of resilient tabs 48, 50 extending distally from opposing first and second circumferential edges of the proximal flange 42a. Alternatively, the distal flange 42b, rather than the proximal flange 42a, may include the locking feature. The resilient tabs 48, 50 have respective hooked distal ends 52, 54 configured to capture opposing circumferential edges of the distal flange 42b to prevent the fluid-transfer assembly 18 from moving out of the approximated position, in which the proximal expandable member 20a is compressed between the proximal and distal flanges 42a, 42b. In some embodiments, the locking feature may alternatively include latches, friction-fit engagements, hook and loop fasteners, clips, hinges, snap-fit engagement, bayonet-type engagements, rotate and lock engagements, or the like.

FIG. 6 illustrates the surgical cannula assembly 10 accessing an underlying cavity, e.g., the abdominal cavity "c". In one methodology, the abdominal cavity "c" is insufflated to establish a pneumoperitoneum. The obturator 100 is positioned within the surgical cannula assembly 10 and the assembled unit is advanced into the abdominal wall "w" while the distal expandable member 20b is in a deflated state such that the proximal expandable member 20a is in an inflated state. Upon positioning the distal expandable member 20b adjacent the abdominal wall "w," a clinician may manually move the proximal and distal flanges 42a, 42b of the fluid-transfer assembly 18 toward one another. Approximation of the proximal and distal flanges 42a, 42b compresses the proximal expandable member 20a therebetween, thereby transferring the inflation medium (e.g., air) disposed within the proximal cavity 38a of the proximal expandable member 20a through the conduits 36 and into the distal cavity 38b of the distal expandable member 20b.

Since the sleeve 16, together with the proximal and distal expandable members 20a, 20b, defines a closed internal volume, as the inflation medium moves from the proximal expandable member 20a to the distal expandable member 20b, the proximal expandable member 20a deflates at the same rate as the distal expandable member 20b inflates. In embodiments, the proximal and distal expandable members 20a, 20b may have different volumes such that they expand and deflate at different rates. Upon expanding the distal expandable member 20b to a desired diameter, the resilient tabs 48, 50 of the proximal flange 42a pass over the lateral sides of the distal flange 42b and the hooks 52, 54 of the resilient tabs 48, 50 ultimately snap-fittingly engage the circumferential edges of the distal flange 42b to selectively lock the proximal and distal flanges 42a, 42b in the approximated position, as shown in FIG. 6.

In the expanded or at least partially expanded condition depicted in FIG. 6, the distal expandable member 20b will resist withdrawal of the surgical cannula assembly 10 from the abdominal cavity "c" while also providing a seal within the internal surface of the abdominal wall "w," minimizing passage of fluids, including inflation fluids, from the abdominal cavity "c".

To withdraw the surgical cannula assembly 10 from the abdominal cavity "c," the resilient tabs 48, 50 of the proximal flange 42a are disengaged from the distal flange 42b and the proximal and distal flanges 42a, 42b are then moved away from one another. As the proximal and distal flanges 42a, 42b of the fluid-transfer assembly 18 are moved toward an expanded state, due to the inflated distal expandable member 20b exhibiting a higher internal pressure than the deflated proximal expandable member 20a, the distal expandable member 20b will transfer the inflation medium from the cavity 38b therein into the proximal expandable member 20a via the conduits 36. As the inflation medium is transferred from the distal expandable member 20b to the proximal expandable member 20a, the gap that forms between the proximal and distal flanges 42a, 42b during their axial separation is filled by the expanding proximal expandable member 42a.

Since the sleeve 16, together with the proximal and distal expandable members 20a, 20b, defines a closed internal volume, as the inflation medium moves from the distal expandable member 20b to the proximal expandable member 20a, the distal expandable member 20b deflates at the same rate at the proximal expandable member 20a inflates. When the distal expandable member 20b is deflated to a selected reduced diameter, the surgical cannula assembly 10 may be withdrawn from the abdominal cavity "c."

With reference to FIGS. 7-9, alternative embodiments of a surgical cannula assembly are illustrated. The surgical cannula assemblies 210, 310 of FIGS. 7-9 are similar to the surgical cannula assembly 10, but for a difference in the associated fluid-transfer assemblies. As such, only features of the fluid-transfer assemblies of the respective surgical cannula assemblies 210, 310 will be described in detail.

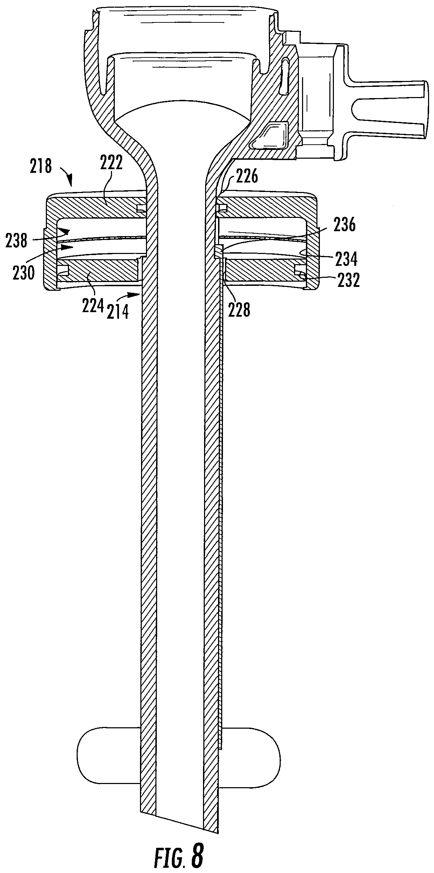

With reference to FIGS. 7 and 8, the surgical cannula assembly 210 generally includes an elongated cannula member 214, an elongate sleeve 216 disposed coaxially about the cannula member 214, and a fluid-transfer assembly 218 mounted to a proximal portion of the sleeve 216. The sleeve 216 has only a distally-located expandable member 220 as opposed to the sleeve 16 of FIGS. 1-6, which has both a proximal and a distal expandable member 20a, 20b. The expandable member 220 may be secured to the distal portion of the cannula member 214 or the sleeve 216 through any of the aforementioned methodologies discussed hereinabove in connection with the outer sleeve 16. In one embodiment, the expandable member 220 is monolithically formed with the distal portion of the outer sleeve 216 from, e.g., a suitable elastomeric material. The expandable member 220 defines an internal chamber therein, and the sleeve 216 defines a longitudinal channel in communication with the internal chamber of the expandable member 220.

The fluid-transfer assembly 218 of the surgical cannula assembly 210 includes a cylindrical member 222 and a piston 224 each of which being disposed about the proximal portion of the sleeve 216 (not shown in FIG. 8). The cylindrical member 222 and the piston 224 define central openings 226, 228 through which the cannula member 214 extends. The piston 224 is longitudinally movable relative to the proximal portion of the cannula member 214, whereas the cylindrical member 222 is longitudinally fixed relative to the proximal portion of the cannula member 214. Alternatively, both of or either one of the cylindrical member 222 and the piston 224 may be movable relative to the cannula member 214. The cylindrical member 222 defines a cavity 230 in which the piston 224 is received. The piston 224 has a seal or gasket 232 disposed thereabout for forming a fluid-tight seal with an inner surface 234 of the cylindrical member 222, while permitting axial movement of the piston 224 within the cavity 230 and relative to the cylindrical member 222.

The inner surface 234 of the cylindrical member 222 may define an annular recess 238 at a proximal location of the cylindrical member 222. The annular recess 238 is dimensioned to releasably receive the piston 224. As such, as the piston 224 moves to a proximal position (i.e., an approximated position of the fluid-transfer assembly 218), the piston 224 moves into the annular recess 236 of the cylindrical member 222 to maintain the fluid-transfer assembly 218 in the approximated position, in which the expandable member 220 is in an inflated state.

The surgical cannula assembly 210 includes a conduit 236 that extends from within the cavity 230 of the fluid-transfer assembly 218 and into the expandable member 220. In one embodiment, the sleeve 216 is disposed about the conduit 236 to seal the conduit 236 with the cannula member 214.

The conduit 236, together with the expandable member 220 and the cavity 230 of the fluid-transfer assembly 218, defines a closed internal volume that contains a fixed amount of inflation medium therein. As such, the expandable member 220 one of expands radially outward or retracts radially inward in response to an axial movement of the plunger 224 within the cavity 230 of the cylindrical member 224. In particular, upon moving the plunger 224 in a proximal direction, the volume of the cavity 230 is reduced, thereby transferring the inflation media (e.g., air, water, gas, etc.) out of the cavity 230 and into the inner chamber of the expandable member 220 via the conduits 236. Alternatively, upon moving the plunger 224 in a distal direction, the volume of the cavity 230 is increased, which temporarily forms a lower pressure region, thereby drawing the inflation medium out of the inner chamber of the expandable member 220 and into the cavity 230 of the fluid-transfer assembly 218 via the conduits 236. In this way, a clinician may control the diameter or overall size of the expandable member 220 by adjusting the position of the plunger 224 of the fluid-transfer assembly 218 without the need of an external/separate fluid source.

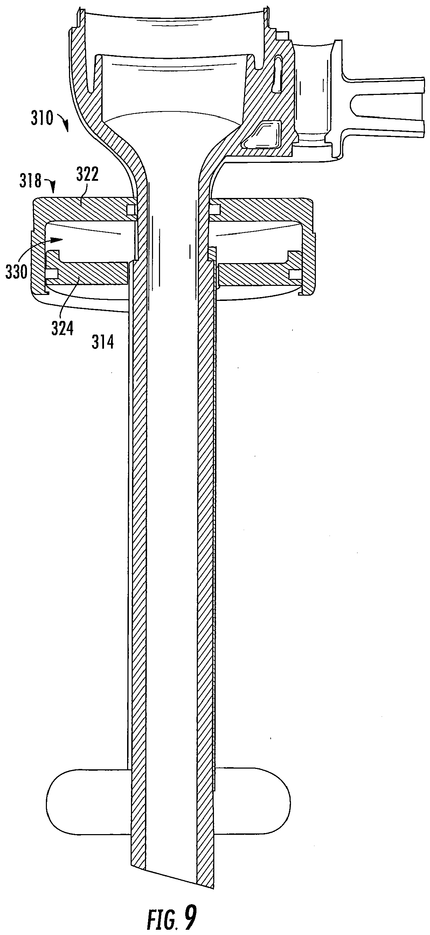

With reference to FIG. 9, another embodiment of a surgical cannula assembly is illustrated 310, which is similar to the surgical cannula assembly 210 of FIGS. 7 and 8. Due to the similarity of the cannula assemblies 210, 310, only selected features of the surgical cannula assembly 310 will be described in detail.

The surgical cannula assembly 310 generally includes an elongated cannula member 314 and an associated fluid transfer assembly 318. The fluid-transfer assembly 318 has a plunger 324 that is rotatably coupled to a cylindrical member 322 rather than being freely axially movable within the cylindrical member 322 as is the plunger 224 of the surgical cannula assembly 210 of FIGS. 7 and 8. The plunger 324 may have a threaded outer surface threadedly coupled to a threaded internal surface of the cylindrical member 322, such that rotation of the plunger 324 relative to the cannula 314 causes the plunger 324 to move axially along the cannula member 314 to transfer inflation media between the cavity 330 of the fluid-transfer assembly 318 and the inner chamber of an expandable member (not shown) disposed at a distal portion of the cannula member 314. In embodiments, the plunger 324 or the cylindrical member 322 may be threadedly coupled to an outer surface of the cannula member 314.

It is contemplated that any of the embodiments of the surgical cannula assemblies 10, 210, or 310 disclosed herein may incorporate either of the fluid-transfer assemblies 18, 218, or 318 described above.

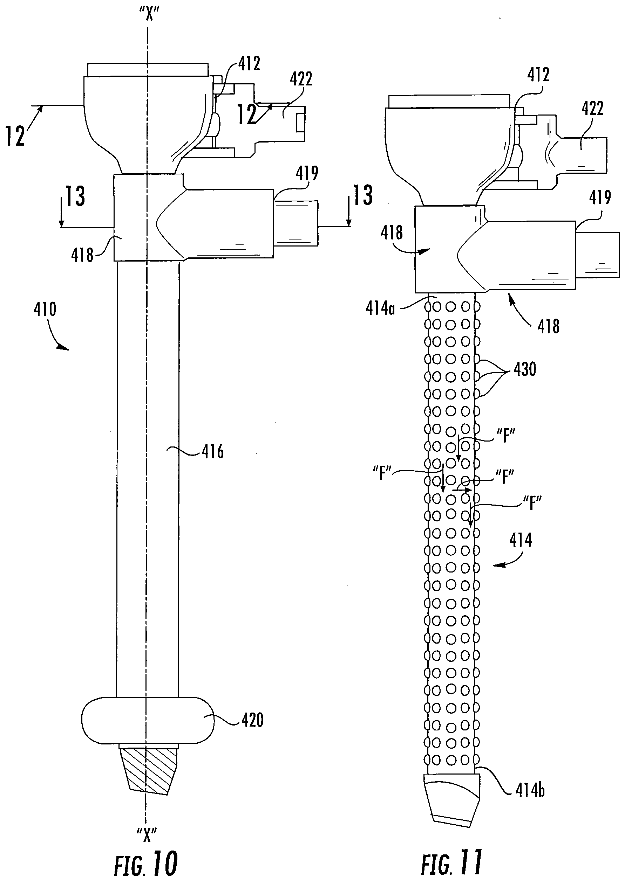

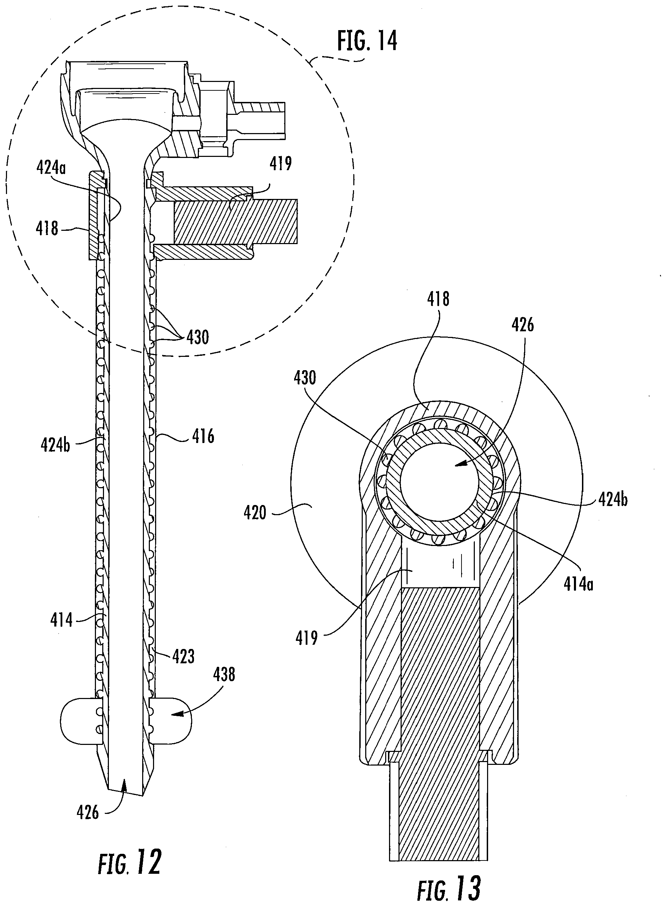

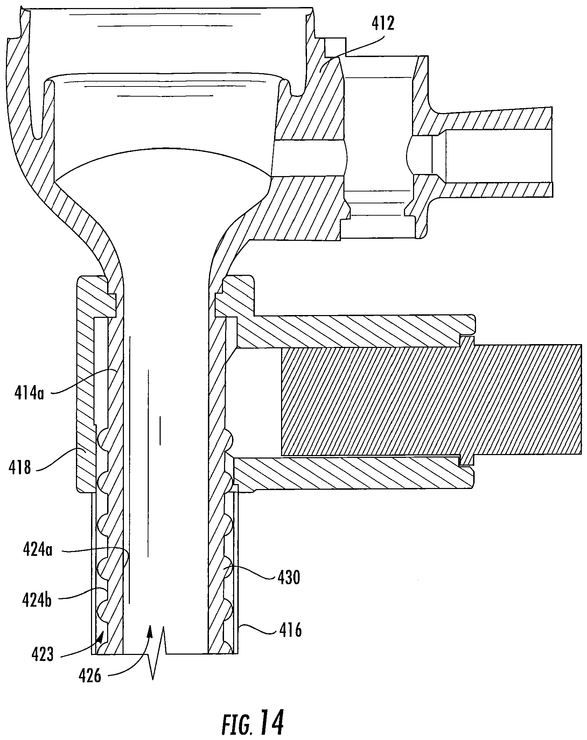

With reference to FIGS. 10-14, yet another embodiment of a surgical cannula assembly 410 is depicted. The surgical cannula assembly 410 includes a cannula housing 412, an elongated cannula member 414 extending distally from the cannula housing 412, an outer sleeve 416 coaxially mounted over the cannula member 414, and a collar 418 coupled to the cannula member 414 at a location adjacent the cannula housing 412. Instead of containing a closed internal volume for the transfer of an inflation medium, the surgical cannula assembly 410 of the present disclosure is configured to be coupled to a fluid source (not shown) via the collar 418 for transferring the inflation medium to an expandable member 420.

The cannula housing 412 is dimensioned for engagement by the clinician and may include one or more internal seals (not shown) adapted to establish a seal about a surgical object introduced therethrough. The cannula housing 412 also may include an insufflation connector 422 (e.g., a luer connector) for connecting to a source of insufflation fluids (not shown) for delivery within, e.g., the abdominal cavity.

The cannula member 414 has proximal and distal portions 414a, 414b, and defines a longitudinal axis "X" along which the cannula member 414 extends. The cannula member 414 has an inner surface 424a defining a longitudinal passageway 426 to permit passage of the surgical object, and an outer surface 424b. The longitudinal passage 426 is also in fluid communication with the insufflation connector 422 to convey insufflation fluids into the abdominal cavity to establish and/or maintain the pneumoperitoneum.

The cannula member 414 includes a plurality of protuberances 430 disposed both circumferentially about the cannula member 414 and along a majority of the length of the cannula member 414. The protuberances 430 are hemispherical, but in some embodiments may assume any suitable shape such as squared, triangular, pyramidal, frusto-conical, elongated, or the like. The protuberances 430 may be arranged randomly on the outer surface 424b of the cannula member 414 or in a defined pattern to define uniform fluid channels along the outer surface 414 of the cannula member. In embodiments, the protuberances 430 may be monolithically formed with the outer surface 424b of the cannula member 414 or, alternatively, attached thereto using adhesives, cements or the like.

The outer sleeve 416 is coaxially mounted about the cannula member 414 and extends from a position within the collar 418 to a position adjacent the distal portion 414b of the cannula member 414. The outer sleeve 416 is spaced from the outer surface 424b of the cannula member 414 by the protuberances 430 to define a plurality of fluid pathways "F" that fluidly interconnect a fluid port 419 and the expandable member 420. The outer sleeve 416 may be secured within the collar 418 and to the cannula member 414 through a friction or interference fit or with the use of adhesives, cements or the like. In one embodiment, the outer sleeve 416 is fabricated from an elastomeric material such as silicone rubber, polyurethane, polyester or the like. Once positioned on the cannula member 414, the outer sleeve 416 is fixed from longitudinal movement, and serves as, e.g., an enclosure enclosing, and optionally sealing, the cannula member 414.

The expandable member 420 is coupled to a distal portion of the outer sleeve 416 and is coaxially mounted about the distal portion 414b of the cannula member 414. The expandable member 420 may be secured to the distal portion of the sleeve 416 through any of the aforementioned methodologies discussed hereinabove in connection with the outer sleeve 16. In an embodiment, the expandable member 420 is secured to the sleeve 416 through an interference or friction fit. The expandable member 420 may be a separate component from the outer sleeve 416 coupled thereto by conventional methodologies. In one embodiment, the expandable member 420 may be monolithically formed with the outer sleeve 416 from, e.g., a suitable elastomeric material. For example, the expandable member 420 may be a segment of the outer sleeve 416, which is subjected to a molding or thermoforming process to be capable of transitioning between unexpanded and expanded conditions. The expandable member 420 defines a cavity or internal chamber 438 therein, and the sleeve 416 defines a longitudinal channel 423 in communication with the cavity 438 of the expandable member 420.

The surgical cannula assembly 410 further includes a collar 418 positioned adjacent the cannula housing 412 and about the proximal portion 414a of the cannula member 414. The collar 418 forms a fluid-tight seal with the proximal portion of the sleeve 416 to facilitate passage of inflation medium into the channel 423 of the sleeve 416 and ultimately into the expandable member 420. The collar 418 includes a fluid port 419 extending laterally relative to the cannula member 414 and configured for coupling to a source of inflation medium. The fluid port 419 is in fluid communication with the fluid pathways "F" defined between the protuberances 430 of the cannula member 414 and the inner surface 424a of the sleeve 424 such that inflation media introduced into the surgical cannula assembly 410 via the fluid port 419 is conveyed by the fluid pathways "F" toward the expandable member 420. As such, the diameter or overall size of the expandable member 420 may be adjusted by selectively transferring inflation media introduced into the cannula assembly 410 via the fluid port 419, through the fluid pathways F" formed by the protuberances 430, and into the expandable member 420.

It is contemplated that any of the cannula members 14, 114, 214, or 314 of the present disclosure may incorporate the protuberances 430 described above.

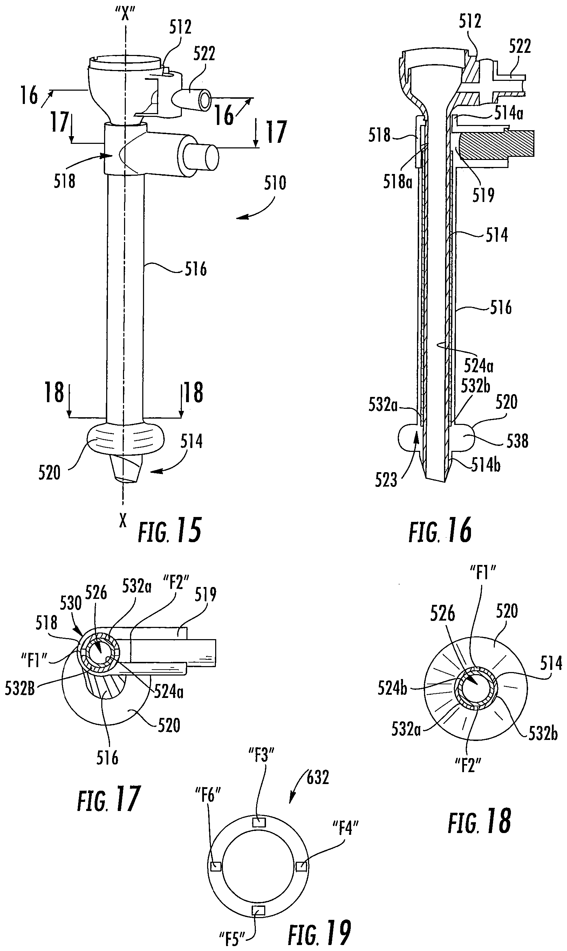

With reference to FIGS. 15-18, yet another embodiment of a surgical cannula assembly 510 is depicted. The surgical cannula assembly 510 includes a cannula housing 512, an elongated cannula member 514 extending distally from the cannula housing 512, an outer sleeve 516 coaxially mounted over the cannula member 514, and a collar 518 coupled to the cannula member 514 adjacent the cannula housing 512.

The cannula housing 512 is dimensioned for engagement by the clinician and may include one or more internal seals (not shown) adapted to establish a seal about a surgical object introduced therethrough. The cannula housing 512 also may include an insufflation connector 522 (e.g., a luer connector) for connecting to a source of insufflation fluids (not shown) for delivery within, e.g., the abdominal cavity.

The cannula member 514 has proximal and distal portions 514a, 514b, and defines a longitudinal axis "X" along which the cannula member 514 extends. The cannula member 514 has an inner surface 524a defining a longitudinal passageway 526 to permit passage of the surgical object, and an outer surface 524b. The longitudinal passageway 526 is also in fluid communication with the insufflation connector 522 to convey insufflation fluids into the abdominal cavity to establish and/or maintain the pneumoperitoneum.

The outer sleeve 516 is coaxially mounted about the cannula member 514 and extends from a position within the collar 518 to a position adjacent the distal portion 514b of the cannula member 514. The outer sleeve 516 may be secured within the collar 518 and to the cannula member 514 through a friction or interference fit or with the use of adhesives, cements or the like. In one embodiment, the outer sleeve 516 is fabricated from an elastomeric material such as silicone rubber, polyurethane, polyester or the like. Once positioned on the cannula member 514, the outer sleeve 516 is fixed from longitudinal movement, and serves as, e.g., an enclosure enclosing, and optionally sealing, the cannula member 514.

The expandable member 520 is coupled to the distal portion of the outer sleeve 516 and is coaxially mounted about the distal portion 514b of the cannula member 514. The expandable member 520 may be secured to the distal portion of the sleeve 516 through any of the aforementioned methodologies discussed hereinabove in connection with the outer sleeve 16. In an embodiment, the expandable member 520 is secured to the sleeve 516 through an interference or friction fit. The expandable member 520 may be a separate component from the outer sleeve 516 coupled thereto by conventional methodologies. In one embodiment, the expandable member 520 may be monolithically formed with the outer sleeve 516 from, e.g., a suitable elastomeric material. For example, the expandable member 520 may be a segment of the outer sleeve 516, which is subjected to a molding or thermoforming process to be capable of transitioning between unexpanded and expanded conditions. The expandable member 520 defines a cavity or internal chamber 538 therein, and the sleeve 516 defines a longitudinal channel 523 in communication with the cavity 538 of the expandable member 528.

The surgical cannula assembly 510 further includes a collar 518 positioned adjacent the cannula housing 512 and about the proximal portion 514a of the cannula member 514. The collar 518 forms a fluid-tight seal with the proximal portion of the sleeve 516 to facilitate passage of inflation medium into the channel 523 of the sleeve 516 and ultimately into the expandable member 520. The inner surface 518a of the collar 518 and the outer surface 524b of the cannula member 514 define an annular slot 530 in which a pair of semi-hemispherical inserts 532a, 532b are received. The inserts 532a, 532b extend longitudinally along the longitudinal axis "X" of the cannula member 514 to maintain the inner surface of the sleeve 516 radially spaced from the outer surface 524b of the cannula member 514 along the majority of the length of the cannula member 514.

The inserts 532a, 532b extend only partially around the circumference of the cannula member 514 to define a pair of fluid pathways "Fl," "F2" between the inserts 532a, 532b. The fluid pathways "Fl," "F2" extend from the collar 518 to the expandable member 520 to permit inflation medium to travel from a fluid port 519 of the collar 518, along the length of the cannula member 514, and into the expandable member 520. As such, the diameter or overall size of the expandable member 520 may be adjusted by selectively transferring inflation media introduced into the surgical cannula assembly 510 via the fluid port 519, through the fluid pathways "Fl," "F2," and into the expandable member 520. Due to the inserts 532a, 532b having structural rigidity, the inserts 532a, 532b prevent the sleeve 516 from collapsing around the cannula member 514, which would block travel of the inflation media through the fluid pathways "Fl," "F2."

With reference to FIG. 19, another embodiment of an insert 632 is illustrated, which is configured for receipt in the annular slot 530 of the surgical cannula assembly 510. In this embodiment, instead of the surgical cannula assembly 510 including a pair of discreet inserts 532a, 532b, the surgical cannula assembly 510 may include one insert 632 fabricated from one piece of extruded material with fluid pathways "F3," "F4," "F5," "F6" formed longitudinally therethrough.

It is contemplated that any of the disclosed embodiments of the surgical cannula assemblies may incorporate either the inserts or the insert described above for spacing the sleeve from the cannula member.

While the inventions have been particularly shown and described with reference to the preferred embodiments, it will be understood by those skilled in the art that various modifications and changes in form and detail may be made therein without departing from the scope of the invention defined in the appended claims.

## Claims

1. A surgical cannula assembly (10), comprising:
an elongated cannula member (14) having proximal and distal portions (14a, 14b) and defining a longitudinal passageway (26) and a longitudinal axis ("X");
a first expandable member (20a) mounted to the proximal portion (14a) of the cannula member;
a second expandable member (20b) mounted to the distal portion (14b) of the cannula member and being in fluid communication with the first expandable member, wherein each of the first and second expandable members defines a cavity (38a, 38b) therein; and
a fluid-transfer assembly (18) including:
a proximal flange (42a) coupled to the proximal portion of the cannula member; and
a distal flange (42b) coupled to the proximal portion of the cannula member, wherein the first expandable member is disposed between the proximal and distal flanges such that movement of at least one of the proximal or distal flanges along the cannula member adjusts a pressure applied by the proximal and distal flanges on the first expandable member to transfer an inflation medium between the first and second expandable members;
**characterised in that** the surgical cannula assembly further comprises an elongated sleeve (16) interconnecting the first and second expandable members, the sleeve disposed about the cannula member and defining a longitudinal channel (30) in fluid communication with the cavity of each of the first and second expandable members, wherein the first and second expandable members (20a, 20b) are monolithically formed with respective proximal and distal portions (16a, 16b) of the sleeve (16).

2. The surgical cannula assembly (10) according to claim 1, wherein the proximal flange (42a) is longitudinally movable relative to the cannula member (14), and the distal flange (42b) is longitudinally fixed relative to the cannula member.

3. The surgical cannula assembly (10) according to claim 2, wherein the proximal flange (42a) is attached to a proximally-oriented surface of the first expandable member (20a), and the distal flange (42b) is attached to a distally-oriented surface of the first expandable member.

4. The surgical cannula assembly (10) according to any preceding claim, wherein the proximal and distal flanges (42a, 42b) are longitudinally spaced from one another along the longitudinal axis ("X") defined by the cannula member (14).

5. The surgical cannula assembly (10) according to any preceding claim, wherein approximation of the proximal and distal flanges (42a, 42b) compresses the first expandable member (20a) therebetween to transfer a portion of the inflation medium from the first expandable member toward the second expandable member (20b), and expansion of the proximal and distal flanges transfers the portion of the inflation medium from the second expandable member toward the first expandable member.

6. The surgical cannula assembly (10) according to any preceding claim, wherein at least one of the proximal or distal flanges (42a, 42b) includes a locking feature configured to releasably attach to the other of the proximal or distal flanges when the proximal and distal flanges are in an approximated position relative to one another.

7. The surgical cannula assembly (10) according to claim 6, wherein the locking feature includes:
a first resilient tab (48) extending from a first side of one of the proximal or distal flanges (42a, 42b); and
a second resilient tab (50) extending from a second side of one of the proximal or distal flanges, the first and second resilient tabs configured to releasably engage one of the proximal or distal flanges when the proximal and distal flanges are in the approximated position.

8. The surgical cannula assembly (10) according to any preceding claim, wherein the cannula member (14) including a plurality of longitudinal ribs (28) extending along the longitudinal axis ("X") defined by the cannula member, adjacent longitudinal ribs defining the longitudinal channel (30) therebetween.

9. The surgical cannula assembly (10) according to claim 9, further comprising a conduit (36) positioned within the longitudinal channel (30) of the cannula member (14), the first expandable member (20a) being in fluid communication with a proximal opening (36a) in the conduit, and the second expandable member (20b) in fluid communication with a distal opening (36b) in the conduit.

10. The surgical cannula assembly (10) according to any of claims 1-7, wherein the cannula member (14) includes a plurality of protuberances (430) disposed both circumferentially about the cannula member and along the longitudinal axis ("X") defined by the cannula member, the plurality of protuberances configured to maintain the sleeve (16) spaced from an outer surface (24b) of the cannula member to define a plurality of fluid pathways that fluidly interconnect the first expandable member (20a) and the second expandable member (20b).

11. The surgical cannula assembly (10) according to any of claims 1-7, further comprising at least one annular insert (532a, 532b, 632) extending along the longitudinal axis ("X") defined by the cannula member (14) and received within an annular slot (530) defined between an outer surface (24b) of the cannula member and an inner surface of the sleeve (16), the annular insert defining at least one fluid pathway that fluidly interconnects the first expandable member (20a) and the second expandable member (20b).

12. The surgical cannula assembly (10) according to any preceding claim, wherein the sleeve (16) is fabricated from an elastomeric material.

13. The surgical cannula assembly (10) according to claim 12, wherein the sleeve (16) has a diameter less than the diameter of the cannula member (14), and is stretch to be positioned about the cannula member.

## Patentansprüche

1. Chirurgische Kanülenanordnung (10), die Folgendes umfasst:
ein längliches Kanülenelement (14), das einen proximalen und einen distalen Abschnitt (14a, 14b) aufweist und einen Längsdurchgang (26) und eine Längsachse ("X") definiert;
ein erstes expandierbares Element (20a), das an dem proximalen Abschnitt (14a) des Kanülenelements montiert ist;
ein zweites expandierbares Element (20b), das an dem distalen Abschnitt (14b) des Kanülenelements montiert ist und in Fluidverbindung mit dem ersten expandierbaren Element steht, wobei jedes des ersten und des zweiten expandierbaren Elements einen Hohlraum (38a, 38b) darin definiert; und
eine Fluidübertragungsanordnung (18), die Folgendes beinhaltet:
einen proximalen Flansch (42a), der mit dem proximalen Abschnitt des Kanülenelements gekoppelt ist; und
einen distalen Flansch (42b), der mit dem proximalen Abschnitt des Kanülenelements gekoppelt ist, wobei das erste expandierbare Element zwischen dem proximalen und dem distalen Flansch derart angeordnet ist, dass eine Bewegung des proximalen und/oder des distalen Flanschs entlang des Kanülenelements einen Druck anpasst, der durch den proximalen und den distalen Flansch an dem ersten expandierbaren Element ausgeübt wird, um ein Aufblasmedium zwischen dem ersten und dem zweiten expandierbaren Element zu übertragen;
**dadurch gekennzeichnet, dass** die chirurgische Kanülenanordnung ferner eine längliche Hülse (16) umfasst, die das erste und das zweite expandierbare Element miteinander verbindet, wobei die Hülse um das Kanülenelement herum angeordnet ist und einen Längskanal (30) definiert, der in Fluidverbindung mit dem Hohlraum jedes des ersten und des zweiten expandierbaren Elements steht, wobei das erste und das zweite expandierbare Element (20a, 20b) monolithisch mit dem jeweiligen proximalen und distalen Abschnitt (16a, 16b) der Hülse (16) ausgebildet sind.

2. Chirurgische Kanülenanordnung (10) nach Anspruch 1, wobei der proximale Flansch (42a) in Längsrichtung relativ zu dem Kanülenelement (14) bewegbar ist und der distale Flansch (42b) in Längsrichtung relativ zu dem Kanülenelement befestigt ist.

3. Chirurgische Kanülenanordnung (10) nach Anspruch 2, wobei der proximale Flansch (42a) an einer proximal ausgerichteten Oberfläche des ersten expandierbaren Elements (20a) angebracht ist und der distale Flansch (42b) an einer distal ausgerichteten Oberfläche des ersten expandierbaren Elements angebracht ist.

4. Chirurgische Kanülenanordnung (10) nach einem der vorhergehenden Ansprüche, wobei der proximale und der distale Flansch (42a, 42b) in Längsrichtung entlang der Längsachse ("X"), die durch das Kanülenelement (14) definiert ist, voneinander beabstandet sind.

5. Chirurgische Kanülenanordnung (10) nach einem der vorhergehenden Ansprüche, wobei eine Annäherung des proximalen und des distalen Flanschs (42a, 42b) das erste expandierbare Element (20a) dazwischen komprimiert, um einen Abschnitt des Aufblasmediums von dem ersten expandierbaren Element in Richtung des zweiten expandierbaren Elements (20b) zu übertragen, und die Expansion des proximalen und des distalen Flanschs den Abschnitt des Aufblasmediums von dem zweiten expandierbaren Element in Richtung des ersten expandierbaren Elements überträgt.

6. Chirurgische Kanülenanordnung (10) nach einem der vorhergehenden Ansprüche, wobei der proximale und/oder der distale Flansch (42a, 42b) ein Verriegelungsmerkmal beinhaltet, das konfiguriert ist, um an dem anderen des proximalen oder des distalen Flanschs lösbar angebracht zu werden, wenn sich der proximale und der distale Flansch in einer angenäherten Position relativ zueinander befinden.

7. Chirurgische Kanülenanordnung (10) nach Anspruch 6, wobei das Verriegelungsmerkmal Folgendes beinhaltet:
eine erste elastische Lasche (48), die sich von einer ersten Seite des proximalen oder des distalen Flanschs (42a, 42b) erstreckt; und
eine zweite elastische Lasche (50), die sich von einer zweiten Seite des proximalen oder des distalen Flanschs erstreckt, wobei die erste und die zweite elastische Lasche konfiguriert sind, um in den proximalen oder den distalen Flansch lösbar einzugreifen, wenn sich der proximale und der distale Flansch in der angenäherten Position befinden.

8. Chirurgische Kanülenanordnung (10) nach einem der vorhergehenden Ansprüche, wobei das Kanülenelement (14) mehrere Längsrippen (28) beinhaltet, die sich entlang der Längsachse (ʺX") erstrecken, die durch das Kanülenelement definiert ist, wobei angrenzende Längsrippen den Längskanal (30) dazwischen definieren.

9. Chirurgische Kanülenanordnung (10) nach Anspruch 9, die ferner eine Leitung (36) umfasst, die innerhalb des Längskanals (30) des Kanülenelements (14) positioniert ist, wobei das erste expandierbare Element (20a) in Fluidverbindung mit einer proximalen Öffnung (36a) in der Leitung steht und das zweite expandierbare Element (20b) in Fluidverbindung mit einer distalen Öffnung (36b) in der Leitung steht.

10. Chirurgische Kanülenanordnung (10) nach einem der Ansprüche 1-7, wobei das Kanülenelement (14) mehrere Vorsprünge (430) beinhaltet, die sowohl in Umfangsrichtung um das Kanülenelement herum als auch entlang der Längsachse ("X") angeordnet sind, die durch das Kanülenelement definiert ist, wobei die mehreren Vorsprünge konfiguriert sind, um die Hülse (16) von einer Außenoberfläche (24b) des Kanülenelements beabstandet zu halten, um mehrere Fluidwege zu definieren, die das erste expandierbare Element (20a) und das zweite expandierbare Element (20b) fluidisch verbinden.

11. Chirurgische Kanülenanordnung (10) nach einem der Ansprüche 1-7, die ferner wenigstens einen ringförmigen Einsatz (532a, 532b, 632) umfasst, der sich entlang der Längsachse (ʺX") erstreckt, die durch das Kanülenelement (14) definiert und innerhalb eines ringförmigen Schlitzes (530) aufgenommen ist, der zwischen einer Außenoberfläche (24b) des Kanülenelements und einer Innenoberfläche der Hülse (16) definiert ist, wobei der ringförmige Einsatz wenigstens einen Fluidweg definiert, der das erste expandierbare Element (20a) und das zweite expandierbare Element (20b) fluidisch verbindet.

12. Chirurgische Kanülenanordnung (10) nach einem der vorhergehenden Ansprüche, wobei die Hülse (16) aus einem elastomeren Material hergestellt ist.

13. Chirurgische Kanülenanordnung (10) nach Anspruch 12, wobei die Hülse (16) einen Durchmesser aufweist, der kleiner als der Durchmesser des Kanülenelements (14) und dehnbar ist, um um das Kanülenelement herum positioniert zu werden.

## Revendications

1. Ensemble canule chirurgicale (10) comprenant :
un élément de canule allongé (14) ayant des parties proximale et distale (14a, 14b) et définissant un passage longitudinal (26) et un axe longitudinal (« X ») ;
un premier élément extensible (20a) monté sur la partie proximale (14a) de l'élément de canule ;
un second élément extensible (20b) monté sur la partie distale (14b) de l'élément de canule et étant en communication fluidique avec le premier élément extensible, chacun des premier et second éléments extensibles définissant une cavité (38a, 38b) à l'intérieur de celui-ci ; et
un ensemble transfert de fluide (18) comportant :
une bride proximale (42a) accouplée à la partie proximale de l'élément de canule ; et
une bride distale (42b) accouplée à la partie proximale de l'élément de canule, le premier élément extensible étant disposé entre les brides proximale et distale de telle sorte que le mouvement d'au moins l'une des brides proximale ou distale le long de l'élément de canule ajuste une pression appliquée par les brides proximale et distale sur le premier élément extensible pour transférer un support de gonflage entre les premier et second éléments extensibles ;
**caractérisé en ce que** l'ensemble canule chirurgicale comprend en outre un manchon allongé (16) interreliant les premier et second éléments extensibles, le manchon étant disposé autour de l'élément de canule et définissant un canal longitudinal (30) en communication fluidique avec la cavité de chacun des premier et des seconds éléments extensibles, les premier et second éléments extensibles (20a, 20b) étant formés de manière monolithique avec les parties proximale et distale respectives (16a, 16b) du manchon (16).

2. Ensemble canule chirurgicale (10) selon la revendication 1, dans lequel la bride proximale (42a) est mobile longitudinalement par rapport à l'élément de canule (14), et la bride distale (42b) est fixée longitudinalement par rapport à l'élément de canule.

3. Ensemble canule chirurgicale (10) selon la revendication 2, dans lequel la bride proximale (42a) est fixée à une surface orientée de manière proximale du premier élément extensible (20a), et la bride distale (42b) est fixée à une surface orientée de manière distale du premier élément extensible.

4. Ensemble canule chirurgicale (10) selon l'une quelconque des revendications précédentes, dans lequel les brides proximale et distale (42a, 42b) sont espacées longitudinalement l'une de l'autre le long de l'axe longitudinal (« X ») défini par l'élément de canule (14).

5. Ensemble canule chirurgicale (10) selon l'une quelconque des revendications précédentes, dans lequel le rapprochement des brides proximale et distale (42a, 42b) comprime le premier élément extensible (20a) entre elles pour transférer une partie du support de gonflage du premier élément extensible vers le second élément extensible (20b), et l'extension des brides proximale et distale transfère la partie du support de gonflage du second élément extensible vers le premier élément extensible.

6. Ensemble canule chirurgicale (10) selon l'une quelconque des revendications précédentes, dans lequel au moins l'une des brides proximale ou distale (42a, 42b) comporte un dispositif de verrouillage conçu pour se fixer de manière amovible à l'autre des brides proximale ou distale lorsque les brides proximale et distale sont dans une position rapprochée l'une par rapport à l'autre.

7. Ensemble canule chirurgicale (10) selon la revendication 6, dans lequel le dispositif de verrouillage comporte :
une première languette élastique (48) s'étendant à partir d'un premier côté de l'une des brides proximale ou distale (42a, 42b) ; et
une seconde languette élastique (50) s'étendant à partir d'un second côté de l'une des brides proximale ou distale, les première et seconde languettes élastiques étant conçues pour entrer en prise de manière amovible avec l'une des brides proximale ou distale lorsque les brides proximale et distale sont dans la position rapprochée.

8. Ensemble canule chirurgicale (10) selon l'une quelconque des revendications précédentes, dans lequel l'élément de canule (14) comporte une pluralité de nervures longitudinales (28) s'étendant le long de l'axe longitudinal (« X ») défini par l'élément de canule, les nervures longitudinales adjacentes définissant un canal longitudinal (30) entre elles.

9. Ensemble canule chirurgicale (10) selon la revendication 9, comprenant en outre un conduit (36) positionné à l'intérieur du canal longitudinal (30) de l'élément de canule (14), le premier élément extensible (20a) étant en communication fluidique avec une ouverture proximale (36a) dans le conduit, et le second élément extensible (20b) étant en communication fluidique avec une ouverture distale (36b) dans le conduit.

10. Ensemble canule chirurgicale (10) selon l'une quelconque des revendications 1 à 7, dans lequel l'élément de canule (14) comporte une pluralité de protubérances (430) disposées à la fois circonférentiellement autour de l'élément de canule et le long de l'axe longitudinal (« X ») défini par l'élément de canule, la pluralité de protubérances étant conçues pour maintenir le manchon (16) espacé d'une surface externe (24b) de l'élément de canule pour définir une pluralité de trajets de fluide qui relient fluidiquement le premier élément extensible (20a) et le second élément extensible (20b).

11. Ensemble canule chirurgicale (10) selon l'une quelconque des revendications 1 à 7, comprenant en outre au moins un insert annulaire (532a, 532b, 632) s'étendant le long de l'axe longitudinal (« X ») défini par l'élément de canule (14) et reçu à l'intérieur d'une fente annulaire (530) définie entre une surface externe (24b) de l'élément de canule et une surface interne du manchon (16), l'insert annulaire définissant au moins un trajet de fluide qui relie fluidiquement le premier élément extensible (20a) et le second élément extensible (20b).

12. Ensemble canule chirurgicale (10) selon l'une quelconque des revendications précédentes, dans lequel le manchon (16) est fabriqué à partir d'un matériau élastomère.

13. Ensemble canule chirurgicale (10) selon la revendication 12, dans lequel le manchon (16) a un diamètre inférieur au diamètre de l'élément de canule (14), et est étiré pour être positionné autour de l'élément de canule.
